# EUROPEAN PATENT SPECIFICATION

(11) **EP 3 106 136 B1**
(45) Date of publication and mention of the grant of the patent: **03.04.2019**
(21) Application number: 16174506.2
(22) Date of filing: 15.06.2016
(51) Int. Cl.: A61F 5/08, A61F 5/56

(54) **NASAL EXPANDER**
NASALE EXPANDER
DILATATEUR NASAL

(30) Priority: 16.06.2015 IT UB20151431
(43) Date of publication of application: 21.12.2016
(73) Proprietor: Bontempelli, Angelo, 31050 Vedelago (TV) (IT)
(72) Inventor: Bontempelli, Angelo, 31050 Vedelago (TV) (IT)
(74) Representative: De Filippis, Sara

(56) References cited:
- CA-A1- 2 618 242
- FR-A1- 2 419 711
- GB-A- 165 537
- US-A- 513 458

## Description

The present invention relates to a nasal spreader device.

In particular, the present invention relates to a device to spread the lateral cartilages of the nose by acting from the inside of the nose.

Nowadays, several types of traditional nasal spreaders are known.

A first type, which acts from the outside of the nose consists of a patch to be applied to the nose so that its opposite ends stick to the nose side walls.

It has a core of elastic material which, in use, tends to spread the side walls so as to widen the nose internal passage section for the air.

A drawback of this is that the spreader is normally single-use or, in any case can be effectively reused a small number of times.

Also, it tends to irritate the skin where it is applied.

A second known type of spreader today is equipped with twin bodies to be inserted through the nostrils into the two nasal cavities.

These twins bodies are joined together by an elastic U-shaped element which mounts on the nasal septum.

By elastic action of the element that joins them, the twins bodies, in use, cause the side walls to diverge from the inside of the nose, to increase the opening of the latter.

This second type of nasal spreader is in plastic material and is quite bulky so that the spreading effect is in part prejudiced by the obstruction that it causes.

A variant of this second kind of nasal spreader is disclosed in patent ES2150374.

This variant is realized with an elastic metallic filament and has a first U-shaped loop, for forking the nasal septum, to which are connected, by means of two auxiliary loops, two second U-shaped loops adapted to house into the nasal passages to act internally between the side walls and the nasal septum for divaricating the nasal ducts.

The first loop and the second loops lie on parallel planes so that the second loops are not aligned with the opening of the nostrils.

This structure has the drawback of being able to cause damage if the nose is hit, for example during sporting activities or due to an involuntary movement during sleep.

Nasal spreaders accrding to the preamble of claim 1 are known from US513458 and CA2618242.

The problem underlying the present invention is to improve both the effectiveness of the respiration worked alongside both safety also making more comfortable the use of a nasal spreader compared to the traditional ones.

Main aim of the present invention is to provide a nasal spreader which provides solution to this problem by solving the drawbacks of the traditional nasal spreaders described above.

Within this aim, it is an aim of the present invention to propose a nasal spreader which is structurally more simple than the known nasal spreaders.

Another object of the present invention is to provide a nasal spreader that is less invasive compared to the known nasal spreaders.

Another object of the invention is to propose a nasal spreader which allows to exert less pressure on the inner walls of the nose, compared to the known nasal spreaders.

This aim, these objects and others which will become apparent hereinafter are achieved by a nasal spreader according to the appended claim 1. Detail features of the nasal spreader according to the invention are disclosed in the dependent claims.

Further characteristics and advantages of the invention will become apparent from the description of a preferred, but not exclusive, embodiment of the nasal spreader according to the invention, illustrated only by way of non-limitative example in the accompanying drawings, in which:
- Figure 1 shows a nasal spreader according to the present invention, seen from above;
- Figure 2 illustrates the nasal spreader of Figure 1 schematically in a use condition;
- Figure 3 illustrates a nasal spreader according to the present invention, seen from above;
- Figure 4 shows a partial sketch view of a nasal spreader according to the present invention.

With particular reference to the above figures, it is indicated with numeral 10 a nasal spreader which, according to the present invention, includes a wire 11 made of an elastically deformable metallic material shaped so as to form two spreader elements 12, 13 and a connecting element 14 that joins them.

Each of the spreader elements 12, 13 comprises:
- a first branch 15 suitable to rest against a side wall A of the user's nose;
- a second branch 16 suitable to rest against a septum wall B of the user's nose;
- a link 17 that connects the branches 15, 16 and is suitable to impart to the latters, in use, a force to spread the side wall A with respect to the septum wall B.

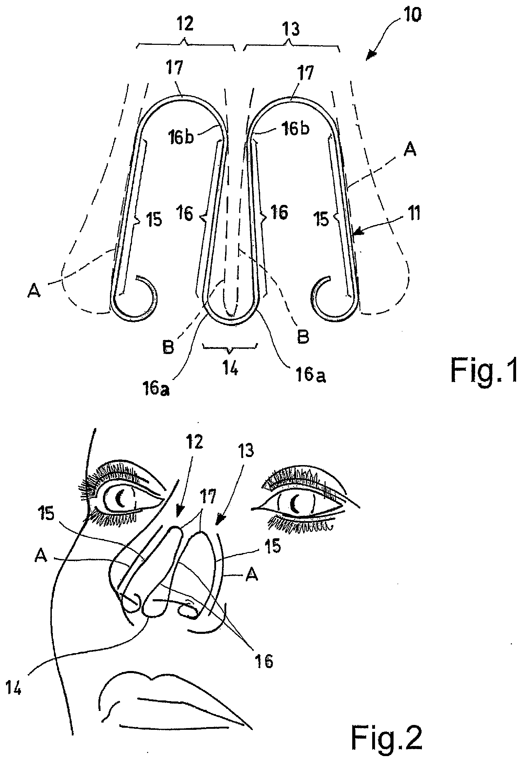

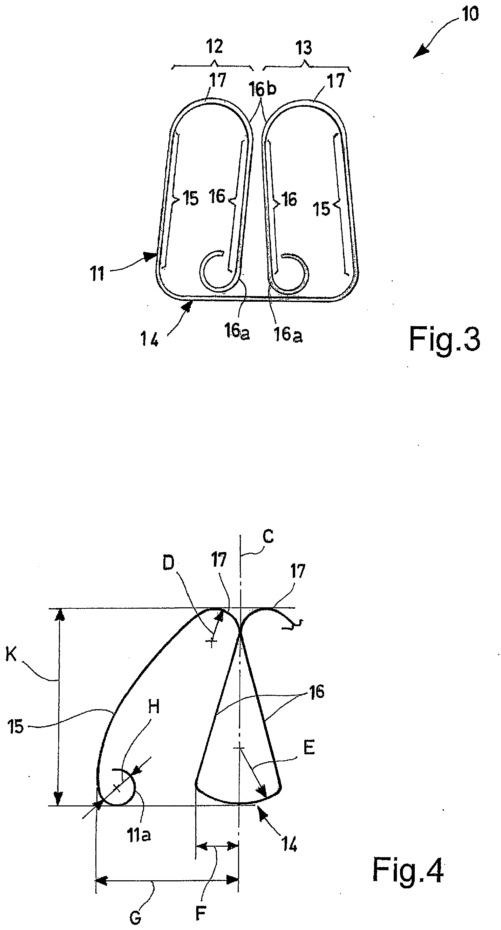

According to the present invention, the spreader elements 12, 13 and the connecting element 14 are spread on a common plane, which for example for the embodiments shown in Figures 1 and 3, coincides with the plane of the sheet.

The nasal spreader 10 according to the present invention is therefore easily insertable and removable from the nose through the nostrils and, developing on a single plane, greatly reduces the risk of damage to the nose in case of impact, compared to nasal spreader in traditional metal wire.

Moreover, it allows to reduce the pressure exerted on the inner walls while allowing to obtain a adequate force for obtaining the desired opening of the nostrils, thanks to the thread-like structure that allows to distribute the opening force along the length of the branches 15, 16 while maintaining a very small bulk so as not to reduce the free section of the nostrils for the air passage of air.

Moreover, it is clear how the nasal spreader 10 according to the present invention is simple to work out and easy to use being formable from a straight wire by shaping it on one plane only.

In a preferred embodiment, the thread 11 is advantageously made of stainless steel and preferably has a diameter between 0.3 and 0.9 mm and more preferably equal to 0.6 mm, so as to obtain an optimal effect of lightness, reduced dimensions and health security.

To obtain a particularly simple structure along with an effective spreading of the nostrils, the branches 15, 16 are preferably substantially straight.

In this case, in use, these branches will bend due to the interference with the walls A and B internal to the nostrils, thus substantially uniformly distributing the spreading stress.

The second branches 16 are preferably mutually convergent in such a way that their mutual distance is greater in correspondence of a first 16a of their ends which, in use, is proximal to the nostrils, and is less in correspondence of their second end 16b that, in use, is distal from the nostrils.

In this way, the nasal spreader 10 is particularly preferred to be employed as it is suitable not to pinch or irritate the edge of the nasal septum, which is particularly sensitive.

A particularly simple to implement and effective structure for limiting the risk of discomfort for the user advantageously provides the links 17 consisting of an arc of a circle or ellipse.

In a first embodiment of the present invention, particularly illustrated by way of non limiting example in figures 1 and 2, the connecting element 14 extends from one end of the second branches 16 of the spreader elements 12, 13 and is adapted to fork, in use, on the user's nasal septum.

In a second embodiment of the present invention, illustrated by way of non-limiting example in Figure 3, the connecting element 14 extends from one of the first terminal branches 15 of the spreader elements 12, 13.

Advantageously, in the described first and second embodiment, the free ends of the wire 11 are shaped so as to avoid coming into contact with mucous membranes, to prevent discomfort or injury to the user, and are preferably wound in a curl.

In a third embodiment, not shown, and which has a particularly solid structure and which allows to use a wire of reduced thickness while maintaining the same elastic modulus of the selected metal material, has said wire which forms a closed loop and wherein the connecting element 14 comprises two portions of said wire, of which:
- A first portion extending from the ends of the second branches of the spreaders elements and is adapted to bridge, in use, on the user's nasal septum;
- a second portion extending from a terminal of the first branches of the spreaders elements.

In particular, it has been found that the use of a nasal spreader according to the present invention brings the following advantages:
- reduction of snoring, until the complete disappearance;
- reduction of sleep apnea, until the complete disappearance;
- the achievement and consolidation of REM during sleep;
- greater and effective sleep quality, greater resting capability;
- increased ability to concentrate and regularization of waking and sleep stages.

Furthermore, the use of a nasal spreader according to the present invention also induces benefits for family members or partners of the user, as a more easily and effectively improved rest of the partners, which enables an improvement in the couple relationship.

Moreover, the use of a nasal spreader according to the invention also allows to improve sports performance even in case of shocks to the nose, thanks to the elasticity of the material thereof.

In addition, the continued use of a nasal spreader according to the invention, leads to a constant expansion and consolidation of the opening of the nasal fossae, which allows to avoid a daily use of the spreader itself. With particular reference to Figure 4, there is illustrated by way of non limiting example a nasal spreader according to the present invention that, in accordance with a preferred variant of the aforementioned first embodiment, has the following peculiarities.

The spreader 10 is symmetrical with respect to a central axis C and, in use, it is aligned to the user's septum.

To obtain an effective restraining action of the spreader 10 to the user's nose, the spreader is configured in such a way that the parts consisting each in a second branch 16 and a link 17, are in contact with each other when the spreader 10 is at rest, i.e. when it is not worn or deformed. Preferably, these two parts are in contact in a connecting portion between the second branch 16 and the link 17.

In general, the spreader 10 illustrated in Figure 4 is in said rest configuration.

To optimize the ergonomics of the spreader 10, this has the following dimensional characteristics:
- the radius of curvature D of the link 17 has a value between 5.5 mm and 7.5 mm;
- the connecting element 14 is curved and preferably has a radius of curvature E of between 8 mm and 11 mm and a width F, in the direction perpendicular to the central axis C, ranging between 6 mm and 7.5 mm;
- the lateral dimensions G of the spreader 10, in a direction perpendicular to the central axis C, is comprised between 16 mm and 22 mm;
- the longitudinal dimension K of the spreader 10, in a direction parallel to the central axis C, is comprised between 21.5 mm and 24 mm;
- the ends 11a of the wire 11 are wound to form a circular curl of 4 mm in diameter.

In more detail, in accordance with the traditional classification anthropometric, the measures described above are preferably defined in accordance with the following table where, for each box wherein are given two numbers, the left number refers to the number of suitable size to a spreader according to the present invention dedicated to a female user and the number of left to a spreader for male user.

The realization of spreaders according to the present invention, dimensioned according to the directions given in the following table, allow to minimize the discomfort for the user and maximize the beneficial effect of the use of the same.

Preferably for child users, i.e. up to 12 years old, the dimensions shown below are reduced by 1/3.

Depending on the specific use and on the kind of user of the spreader, the diameter of the wire 11 will be chosen between 0.6 mm and 0.5 mm, this second measure being in particular suitable to spreaders for use by children.

| | Cathegory caucasian/african | Cathegory african/african | Cathegory asiatic/african |
|---|---|---|---|
| D [mm] | 5,5-5,8 | 6-7,5 | 6-6,5 |
| E [mm] | 8-10,5 | 8-10 | 9-11 |
| F [mm] | 6-7 | 6,5-7,5 | 6,5-6 |
| G [mm] | 16-17 | 19,5-22 | 19-19,5 |
| H [mm] | 4 | 4 | 4 |
| K [mm] | 22,7-24 | 21,5-22,7 | 22,5-24 |

It should be noted that a further advantage of a nasal spreader according to the present invention is that, in use, it rests on internal tissues to the user's nose that are less sensitive than those on which the conventional spreaders rest, that are on the central portion of the side walls of the nostrils.

In fact, a nasal spreader according to the present invention rests on the basis of the curve of the wall side of the nostril, allowing a more extended use with less discomfort to the user.

For sensitivity it is intended not only the attitude of the tissue to detect a tactile stimulus, but also the attitude of the tissue to react, for example with an irritation state to an interference with a foreign body irrespective of the existence of a tactile perception.

Given that the olfactory functionality resides mainly in correspondence of the cartilages of the outer walls of the nose, a spreader according to the invention, in use, by avoiding the contact with these anatomical parts by virtue of its structural configuration, allows to prevent to compromise such olfactory functionality or reduce the sensitivity of these parts.

The invention thus conceived is susceptible to numerous modifications and variants, all falling within the protective scope of the appended claims. Moreover, all the details may be replaced with other technically equivalent elements.

In practice, the materials employed, as well as the shapes and the dimensions, may be varied depending on the contingent requirements and state of the art.

Where the constructional features and techniques mentioned in the following claims are followed by reference signs or numbers, such signs and reference numbers have been indicated with the sole purpose of increasing the intelligibility of the claims and consequently, they do not constitute in any way a limitation to the interpretation of each element identified, purely by way of example, by such signs and reference numbers.

## Claims

1. Nasal spreader comprising a metallic material wire (11) which is elastically deformable and shaped so as to form two connected spreader elements (12, 13) and a connecting element (14) that connects said spreader elements (12, 13) to each other, each of said spreader elements (12, 13) comprising:
- a first branch (15) configured to rest against a side wall (A) of the user's nose;
- a second branch (16) configured to rest against a wall of the septum (B) of the user's nose
each of said first and second branches (15, 16) comprises a first end and a second end;
- a link (17) connecting the respective first ends of said branches (15, 16) of each of said spreader elements (12, 13) and that is configured to impart to the latters a force for, in use, to open out the lateral wall (A) with respect to the wall of the septum (B); said spreader elements (12, 13) and said connecting element (14) extending on the same plane;
**characterized in that**:
- said wire (11) forms a closed loop and said connecting element (14) comprises two portions of said wire (11), a first portion of said portions extends from and connects the second ends of the respective second branches (16) of said spreader elements (12, 13) and is adapted to mount, in use, on the nasal septum and a second portion of said portions extends from and connects the second ends of the respective first branches (15) of said spreader elements (12, 13);
or
- said connecting element (14) extends from and connects the second ends of the respective second branches (16) of said spreader elements (12, 13) and is adapted to mount, in use, on the nasal septum of the user; wherein said nasal spreader (10) is symmetrical with respect to a central axis (C) which in use is aligned with the septum of the nose and that said link (17) has a radius of curvature (D) of a value comprised between 5.5 mm and 7.5 mm; said connecting element (14) is curved and has a radius of curvature (E) comprised between 8 mm and 11 mm and has an overall dimension (F), in a direction perpendicular to said central axis (C), extending between 6 mm and 7.5 mm; said nasal spreader (10) has a side dimension (G), in the direction perpendicular to the central axis (C), extending is between 16 mm and 22 mm; said nasal spreader (10) has a longitudinal dimension (K), in a direction parallel to said central axis (C) extending between 21.5 mm and 24 mm; said wire (11) has ends (11a) each bent to form a circular curl of a diameter of 4 mm;
or
- said connecting element (14) extends from and connects the second ends of the respective first branches (15) of said spreader element (12, 13).

2. Nasal spreader according to claim 1 **characterized in that** said wire (11) is made up of stainless steel.

3. Nasal spreader according to claim 2 **characterized in that** said wire (11) has a section diameter length comprised between 0.3 and 0.9 mm and preferably equal to 0.6 mm.

4. Nasal spreader according to one of the preceding claims, **characterized in that** said branches (15, 16) are substantially straight.

5. Nasal spreader according to one of the preceding claims **characterized in that** said second branches (15, 16) are convergent relative to one another in such a way that their mutual distance is greater in correspondence to one of their first end that, in use, is proximal to the nostrils, and is lower in correspondence of one of their second end that, in use, is distal from the nostrils.

6. Nasal spreader according to one of the preceding claims, **characterized in that** each of said links consist of an arc of a circle or ellipse.

## Patentansprüche

1. Nasenspreizer, umfassend einen Draht (11) aus metallischem Material, der elastisch verformbar und so geformt ist, dass er zwei verbundene Spreizelemente (12, 13) bildet, und ein Verbindungselement (14), das die Spreizelemente (12, 13) miteinander verbindet, wobei jedes der Spreizelemente (12, 13) umfasst:
- einen ersten Abzweig (15), der konfiguriert ist, um an einer Seitenwand (A) der Nase des Benutzers aufzuliegen;
- einen zweiten Abzweig (16), der konfiguriert ist, um an einer Wand des Septums (B) der Nase des Benutzers anzuliegen, wobei jeder der ersten und zweiten Abzweige (15, 16) ein erstes Ende und ein zweites Ende umfasst;
- eine Verbindung (17), die die jeweiligen ersten Enden der Abzweige (15, 16) jedes der Spreizelemente (12, 13) verbindet und die konfiguriert ist, um den Letzteren eine Kraft zu verleihen, um im Gebrauch die Seitenwand (A) in Bezug auf die Wand des Septums (B) zu öffnen; wobei sich die Spreizelemente (12, 13) und das Verbindungselement (14) auf derselben Ebene erstrecken;
**dadurch gekennzeichnet, dass**:
- der Draht (11) eine geschlossene Schlaufe bildet und das Verbindungselement (14) zwei Abschnitte des Drahtes (11) umfasst, wobei sich ein erster Abschnitt der Abschnitte von den zweiten Enden der jeweiligen zweiten Abzweige (16) der Spreizelemente (12, 13) erstreckt und diese verbindet und geeignet ist, bei Gebrauch an der Nasenscheidewand befestigt zu werden, und ein zweiter Abschnitt der Abschnitte sich von den zweiten Enden der jeweiligen erste Abzweige (15) der Spreizelemente (12, 13) erstreckt und diese verbindet; oder
- das Verbindungselement (14) sich von den zweiten Enden der jeweiligen zweiten Abzweige (16) der Spreizelemente (12, 13) erstreckt und diese verbindet und geeignet ist, bei Gebrauch an der Nasenscheidewand des Benutzers befestigt zu werden; wobei der Nasenspreizer (10) symmetrisch in Bezug auf eine Mittelachse (C) ist, die bei Gebrauch mit der Nasenscheidewand ausgerichtet ist, und dass die Verbindung (17) einen Krümmungsradius (D) von einem Wert zwischen 5,5 mm und 7,5 mm aufweist; das Verbindungselement (14) gebogen ist und einen Krümmungsradius (E) zwischen 8 mm und 11 mm aufweist und eine Gesamtabmessung (F) in einer Richtung senkrecht zur Mittelachse (C) aufweist, die sich zwischen 6 mm und 7,5 mm erstreckt; der Nasenspreizer (10) eine Seitenabmessung (G) in der Richtung senkrecht zur Mittelachse (C) aufweist, die sich zwischen 16 mm und 22 mm erstreckt; der Nasenspreizer (10) eine Längsabmessung (K) in einer Richtung parallel zur Mittelachse (C) aufweist, die sich zwischen 21,5 mm und 24 mm erstreckt; der Draht (11) Enden (11a) aufweist, die jeweils gebogen sind, um eine kreisförmige Volute mit einem Durchmesser von 4 mm zu bilden; oder
- das Verbindungselement (14) sich von den zweiten Enden der jeweiligen ersten Abzweige (15) des Spreizelements (12, 13) erstreckt und diese verbindet.

2. Nasenspreizer nach Anspruch 1, **dadurch gekennzeichnet, dass** der Draht (11) aus Edelstahl besteht.

3. Nasenspreizer nach Anspruch 2, **dadurch gekennzeichnet, dass** der Draht (11) eine Profildurchmesserlänge zwischen 0,3 und 0,9 mm und vorzugsweise gleich 0,6 mm aufweist.

4. Nasenspreizer nach einem der vorstehenden Ansprüche, **dadurch gekennzeichnet, dass** die Abzweige (15, 16) im Wesentlichen gerade sind.

5. Nasenspreizer nach einem der vorstehenden Ansprüche, **dadurch gekennzeichnet, dass** die zweiten Abzweige (15, 16) relativ zueinander in einer Weise konvergent sind, so dass ihr gegenseitiger Abstand größer entsprechend einem von ihren ersten Enden ist, das bei Gebrauch proximal zu den Nasenlöchern liegt und niedriger entsprechend von einem ihrer zweiten Enden ist, das bei Gebrauch distal zu den Nasenlöchern liegt.

6. Nasenspreizer nach einem der vorstehenden Ansprüche, **dadurch gekennzeichnet, dass** jede der Verbindungen aus einem Bogen von einem Kreis oder einer Ellipse besteht.

## Revendications

1. Écarteur nasal comprenant un fil en matériau métallique (11) qui est élastiquement déformable et dont la forme permet de former deux éléments d'écarteur reliés (12, 13) et un élément de liaison (14) reliant lesdits éléments d'écarteur (12, 13) l'un à l'autre, chacun desdits éléments d'écarteur (12, 13) comprenant :
- une première branche (15) configurée pour reposer contre une paroi latérale (A) du nez de l'utilisateur ;
- une seconde branche (16) configurée pour reposer contre une paroi du septum (B) du nez de l'utilisateur; chacune desdites première et seconde branches (15, 16) comprenant une première extrémité et une seconde extrémité ;
- une liaison (17) reliant les premières extrémités respectives desdites branches (15, 16) de chacun desdits éléments d'écarteur (12, 13) et qui est configurée pour communiquer aux derniers une force pour, en cours d'utilisation, ouvrir vers l'extérieur la paroi latérale (A) par rapport à la paroi du septum (B) ; lesdits éléments d'écarteur (12, 13) et ledit élément de liaison (14) s'étendant sur le même plan ;
**caractérisé en ce que** :
- ledit fil (11) forme une boucle fermée et ledit élément de liaison (14) comprend deux parties dudit fil (11), une première partie desdites parties s'étend depuis et relie les secondes extrémités des secondes branches respectives (16) desdits éléments d'écarteur (12, 13) et est adaptée à être montée, en cours d'utilisation, sur le septum nasal et une seconde partie desdites parties s'étend depuis et relie les secondes extrémités des premières branches respectives (15) desdits éléments d'écarteur (12, 13) ;
ou
- ledit élément de liaison (14) s'étend depuis et relie les secondes extrémités des secondes branches respectives (16) desdits éléments d'écarteur (12, 13) et est adapté à être monté, en cours d'utilisation, sur le septum nasal de l'utilisateur ; dans lequel ledit écarteur nasal (10) est symétrique par rapport à un axe central (C) qui, en cours d'utilisation, est aligné avec le septum du nez et **en ce que** ladite liaison (17) a un rayon de courbure (D) ayant une valeur comprise entre 5,5 mm et 7,5 mm ; ledit élément de liaison (14) est courbe et a un rayon de courbure (E) compris entre 8 mm et 11 mm et a une dimension globale (F), dans une direction perpendiculaire audit axe central (C), s'étendant entre 6 mm et 7,5 mm ; ledit écarteur nasal (10) a une dimension latérale (G), dans la direction perpendiculaire à l'axe central (C), comprise entre 16 mm et 22 mm ; ledit écarteur nasal (10) a une dimension longitudinale (K), dans une direction parallèle audit axe central (C), comprise entre 21,5 mm et 24 mm ; ledit fil (11) a des extrémités (11a) pliées chacune pour former une boucle circulaire d'un diamètre de 4 mm ;
ou
- ledit élément de liaison (14) s'étend depuis et relie les secondes extrémités des premières branches respectives (15) dudit élément d'écarteur (12, 13).

2. Écarteur nasal selon la revendication 1 **caractérisé en ce que** ledit fil (11) est en acier inoxydable.

3. Écarteur nasal selon la revendication 2 **caractérisé en ce que** ledit fil (11) a une longueur de diamètre de section comprise entre 0,3 et 0,9 mm et de préférence égale à 0,6 mm.

4. Écarteur nasal selon l'une des revendications précédentes, **caractérisé en ce que** lesdites branches (15, 16) sont sensiblement droites.

5. Écarteur nasal selon l'une des revendications précédentes, **caractérisé en ce que** lesdites secondes branches (15, 16) convergent l'une par rapport à l'autre de telle sorte que leur distance mutuelle est plus grande en correspondance avec l'une de leurs premières extrémités qui, en cours d'utilisation, est proximale des narines, et est plus petite en correspondance de l'une de leurs secondes extrémités qui, en cours d'utilisation, est distale des narines.

6. Écarteur nasal selon l'une des revendications précédentes, **caractérisé en ce que** chacun desdites liaisons consiste en un arc de cercle ou une ellipse.
